(19) Europäisches Patentamt
European Patent Office
Office européen des brevets

(11) Veröffentlichungsnummer: **0 032 670**
**B1**

(12) **EUROPÄISCHE PATENTSCHRIFT**

(45) Veröffentlichungstag der Patentschrift: **13.04.83**

(21) Anmeldenummer: **81100061.1**

(22) Anmeldetag: **08.01.81**

(51) Int. Cl.³: **C 09 K 11/06**, C 07 D 311/08, C 09 B 57/02 // H01S3/20, H01L31/04

(54) **Lichtsammelsysteme und die Verwendung von Cumarinderivaten als Energiewandler in ihnen.**

(30) Priorität: **19.01.80 DE 3001877**

(43) Veröffentlichungstag der Anmeldung: **29.07.81 Patentblatt 81/30**

(45) Bekanntmachung des Hinweises auf die Patenterteilung: **13.04.83 Patentblatt 83/15**

(84) Benannte Vertragsstaaten: **CH DE FR GB IT LI NL**

(56) Entgegenhaltungen:
**CH-A-428 953**
**DE-A-2 411 969**
**DE-A-2 837 257**
**DE-B-1 764 982**
**FR-A-1 338 020**
**GB-A-2 017 133**

(73) Patentinhaber: **BAYER AG, Zentralbereich Patente, Marken und Lizenzen, D-5090 Leverkusen 1, Bayerwerk (DE)**

(72) Erfinder: **Arndt, Frank, Dr., Buschstrasse 171, D-4150 Krefeld (DE)**
Erfinder: **Claussen, Uwe, Dr., Carl-Rumpff-Strasse 29, D-5090 Leverkusen (DE)**
Erfinder: **Harnisch, Horst, Dr., Heinenbusch 4, D-5203 Much (DE)**
Erfinder: **Schellhammer, Carl-Wolfgang, Dr., Katharinenthal 26, D-5060 Bergisch-Gladbach 2 (DE)**

EP 0 032 670 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Lichtsammelsysteme und die Verwendung von Cumarinderivaten als Energiewandler in ihnen

Vorrichtungen zum Sammeln diffuser elektromagnetischer Strahlung durch Ausnützen der Fluoreszenz sind bekannt. Kernstück der Sammler ist ein gegenüber der Umgebung optisch dichteres Medium, das fluoreszenzfähige Zentren enthält (DE-A 2 620 115).

Die ökonomische Verwendbarkeit eines Lichtsammelsystems wird in hohem Maße von der Brauchbarkeit des in ihm als Lichtwandler eingesetzten Farbstoffs bestimmt. An die optische Qualität dieses Farbstoffs werden extreme Anforderungen gestellt [Appl. Phys. 14 123 – 139 (1977)].

Die Erfindung betrifft neue Lichtsammelsysteme, die dadurch gekennzeichnet sind, daß sie als Energiewandler ein Cumarinderivat der Formel

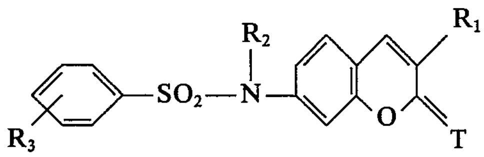

(I)

enthalten.

In Formel (I) bedeuten:

T O oder $NR_4$,

wobei

$R_4$ für Wasserstoff, gegebenenfalls substituiertes Alkyl oder gegebenenfalls substituiertes Aryl steht;

$R_1$ ein über ein C-Atom gebundener carbo- oder heterocyclischer sauerstofffreier 5- oder 6gliedriger Ring oder ein über ein N-Atom gebundener 5- oder 6gliedriger heterocyclischer Ring, der zu einer durch die Cumarin-N-Heterocyclus-Bindung gelegten Achse rotationsunsymmetrisch ist, wobei die genannten 5- oder 6gliedrigen Ringe nicht-ionogene Substituenten tragen können und an sie ein gegebenenfalls substituierter Benzol- oder ein gegebenenfalls substituierter Naphthalring ankondensiert sein kann;

$R_2$ Wasserstoff, Alkyl, Cycloalkyl, Aralkyl, Aryl, wobei die genannten Kohlenwasserstoffreste substituiert sein können und

$R_3$ Wasserstoff, Alkyl, Cycloalkyl, Aralkyl, Aryl oder einen Rest $-OR_5$, $-SO_2R_5$, $-SO_2NHR_5$, $-NHCOR_5$, $COOR_5$ oder Halogen bezeichnen, wobei $R_5$ für Alkyl, Cycloalkyl, Aralkyl oder Aryl steht.

$R_1$ steht bevorzugt für einen 5- oder 6gliedrigen heterocyclischen Ring, der 1, 2 oder 3 Heteroatome N oder S enthält und an den ein Benzolring ankondensiert sein kann, wobei sowohl der heterocyclische Ring wie auch der ankondensierte Benzolring durch beispielsweise Alkyl, Aryl, Aralkyl, Cycloalkyl, Halogen, Alkoxy, Cyan und Acyl substituiert sein können.

Als Beispiele für $R_1$ in der Bedeutung eines 5- oder 6gliedrigen heterocyclischen Ringes an den ein Benzolring ankondensiert sein kann, seien genannt: Pyrazol, Imidazol, Thiazol, 1.2.4-Triazol, 1.3.4-Thiadiazol, Benzimidazol, Benzthiazol, Pyridin, Benzpyrimidon.

Besonders bevorzugt steht $R_1$ für einen heterocyclischen Rest der Formel

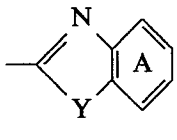

(II)

in der

Y $NR_6$ oder S bezeichnet, wobei

$R_6$ für Alkyl, Aryl, Cycloalkyl oder Aralkyl steht und der Ring

A durch Alkyl, Alkylsulfonyl, Aryl, Aralkyl, Cycloalkyl, Halogen, Alkoxy, Cyan und Acyl substituiert sein kann.

Alkyl ($R_1$, $R_2$, $R_3$, $R_4$, $R_5$, $R_6$, A) steht bevorzugt für $C_1-C_6$-Alkyl.

Aryl ($R_1$, $R_2$, $R_3$, $R_4$, $R_5$, $R_6$, A) steht bevorzugt für Phenyl.

Cycloalkyl ($R_1$, $R_2$, $R_3$, $R_5$, $R_6$, A) steht bevorzugt für $C_3-C_7$-Cycloalkyl insbesondere Cyclopentyl und Cyclohexyl.

Aralkyl ($R_1$, $R_2$, $R_3$, $R_5$, $R_6$, A) steht bevorzugt für Benzyl und Phenethyl.

Acyl ($R_1$, A) steht bevorzugt für ($C_1-C_6$-Alkyl)carbonyl, Benzoyl, $C_1-C_6$-Alkylsulfonyl und Phenylsulfonyl.

Halogen ($R_1$, A) steht bevorzugt für Chlor, Brom und Fluor.

Alkoxy ($R_1$, A) steht bevorzugt für $C_1-C_6$-Alkoxy. Substituiertes Alkyl ($R_2$, $R_4$) steht für Alkyl, das beispielsweise durch Halogen wie Chlor und Brom, Cyan, Trifluormethyl, $C_1-C_6$-Alkoxy, $C_1-C_6$-Alkylsulfonyl, Phenylsulfonyl, durch $C_1-C_6$-Alkyl und Phenyl mono- und disubstituiertes Carbamoyl, Carbamoyl, Sulfamoyl, durch $C_1-C_6$-Alkyl und Phenyl mono- und disubstituiertes Amino, substituiert sein kann.

Substituiertes Aryl ($R_2$, $R_4$), substituiertes Cycloalkyl ($R_2$) und substituiertes Aralkyl ($R_2$) stehen für Kohlenwasserstoffreste, die neben den vorstehend für Alkyl genannten Substituenten beispielsweise noch durch $C_1-C_6$-Alkyl substituiert sein können.

Weitere bevorzugt als Lichtwandler verwendbare Verbindungen sind Cumarinderivate der Formel

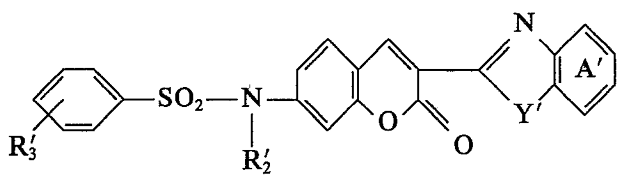

(III)

in der

$R_2'$ Wasserstoff, $C_1-C_4$-Alkyl, insbesondere Methyl und Ethyl,

$R_3'$ Wasserstoff, $C_1-C_4$-Alkyl, insbesondere Methyl und Ethyl, $C_1-C_4$-Alkylsulfonyl, Phenylsulfonyl, $C_1-C_4$-Alkoxy, Phenoxy

Y' $NR_6'$ oder S bezeichnen, wobei

$R_6'$ $C_1-C_4$-Alkyl, insbesondere Methyl, oder Phenyl bezeichnet und wobei der Ring

A' durch $C_1-C_4$-Alkyl, insbesondere Methyl, $C_1-C_4$-Alkoxy, insbesondere Methoxy und Halogen, insbesondere Chlor, substituiert sein kann.

Die erfindungsgemäß als Energiewandler in Lichtsammelsystemen verwendbaren Verbindungen der Formel (I) sind bekannt (s. z. B. die deutschen Offenlegungsschrift 2 702 237) oder können in Analogie zu literaturbekannten Verfahren hergestellt werden.

Die neuen Lichtsammelsysteme, bei denen es sich um Formkörper geeigneter Geometrie, d. h. optische Systeme handelt, bei denen das Verhältnis von Emissions- zu Absorptionsfläche 1 : 50 bis 1 : 2000 betragen kann, sind geeignet, einfallende diffuse elektromagnetische Strahlung zu absorbieren und sie in einem gegenüber der Umgebung optisch dichteren Medium nahezu verlustlos zu emittieren, wodurch der Hauptteil des Emissionslichtes total reflektiert im Medium verbleibt.

Nur der Anteil des emittierten Lichts, dessen Emissionsbande weitgehend frei von Absorption ist, ist für den erfindungsgemäßen Zweck nutzbar.

Daher ist es zweckmäßig, die Farbstoffe der Formel (I) vor Einsatz in die Lichtsammelsysteme besonders zu reinigen. Insbesondere müssen sie weitgehend frei sein von langwellig absorbierenden Verunreinigungen.

Die Erfindung betrifft demnach bevorzugt Lichtsammelsysteme, die dadurch gekennzeichnet sind, daß sie einen Farbstoff der Formel (I) enthalten, der in einer 0,05%igen Lösung, beispielsweise in Chloroform, gemessen in einer Schichtdicke von 10 cm, einen Anstieg der Transmission von 0% auf >90% bei einer Änderung der Wellenlänge von 25—100 nm, vorzugsweise 25—45 nm aufweisen.

Die Reinigung der Farbstoffe kann in günstigen Fällen durch mehrfache sorgfältige Kristallisation erfolgen. Zumeist ist eine säulenchromatographische Trennung an festen Träger wie $Al_2O_3$ oder $SiO_2$ erforderlich. Besonders schonend erhält man hohe Reinheitsgrade verteilungschromatographisch, z. B. durch Steady-State-(O'Keefe)- oder Gegenstromverteilung nach Craig.

Die neuen Lichtsammelsysteme können z. B. in Verbindung mit Solarzellen zur Nutzbarmachung der Sonnenenergie und in Szintillatoren bekannter Art [s. z. B. J. B. Birks: The Theory and Practice of Scintillation Counting (Pergamon Press, London 1964); J. Opt. Am. 39, 912 (1949); J. Appl. Phys. 40, 3544 (1969); Nuclear Instruments a. Methods 87, 111—123 (1970); Research Disclosure, S. 43 (1977); DE-OS 2 629 641] Verwendung finden. Darüber hinaus eignen sie sich in Verbindung mit elektronischen Steuerungen als Anzeigevorrichtungen mit sehr geringem Energieverbrauch, und weiterhin eignen sie sich ohne elektronische Bauteile für vielerlei Anzeige-, Hinweis- und Markierungszwecke, z. B. in passiven Anzeigeelementen, Hinweis- und Verkehrszeichen wie Ampeln.

Die erfindungsgemäßen Lichtsammelsysteme enthalten den Farbstoff in einer Flüssigkeit oder einem Festkörper gelöst, wobei je nach Einsatzgebiet des Lichtsammelsystems, verschiedenste geometrische Formen in Frage kommen. Geeignete feste Medien wie sie z. B. zum Sammeln von Licht in Verbindung mit Solarzellen und in passiven Anzeigeelementen eingesetzt werden, sind z. B. lichtdurchlässige, optisch verwendbare Kunststoffe wie Homo- und Copolymerisate der Acrylsäure(derivate) oder Polycarbonate. Des weiteren können die Lichtsammelsysteme den Farbstoff auch in

einer Flüssigkeit — z. B. Alkohol, Keton, Halogenkohlenwasserstoff, Ether — gelöst enthalten. Gut geeignete Lösungsmittel sind z. B. Ethanol, Propanol, Methyl-ethyl-keton, Aceton, Cyclohexanon, Chloroform, Perchlorethylen, Glykolmonomethylether.

Die Verwendung der Farbstoffe der Formel (I) in Feststoffen ist bevorzugt.

Die erfindungsgemäße Verwendung der Farbstoffe der Formel (I) ist in hohem Maße vorteilhaft, da sie neben einer guten Quantenausbeute und einem hohen Verstärkungsfaktor ausgezeichnete Lichtechtheit aufweisen und damit eine ökonomische Verwendbarkeit der neuen Lichtsammelsysteme gewährleisten.

Es muß als überraschend bezeichnet werden, daß die Farbstoffe der Formel (I) sich zur vorteilhaften Verwendung in Lichtsammelsystemen eignen, da zahlreiche stark fluoreszierende Farbstoffe wie z. B. Rhodamin G nicht brauchbar sind. Ebenso sind die schon sehr weitgehenden Forderungen, die an die optische Qualität von Laserfarbstoffen gestellt werden, in vielen Fällen nicht ausreichend, um die Verwendung dieser Farbstoffe in Lichtsammelsystemen zu empfehlen.

## Beispiel 1

4 g 3-Benzthiazolyl-7-phenylsulfonylaminocumarin werden in 60 ml Chloroform gelöst und an 800 g Kieselgel (Merck) chromatographiert. Elutionsmittel ist Essigester/Methanol 3 : 1. Man erhält 3,2 g Produkt, das aus n-Butanol umkristallisiert wird.

500 mg der Verbindung werden in 1 Liter $CHCl_3$ gelöst und in einer Schichtdicke von 10 cm die Transmission T gemessen. Die Durchlässigkeit beträgt 0% bei 449 nm und bei 479 nm 93%. Die Fluoreszenzquantenausbeute $\Phi$ beträgt 0,87, der Stokes-Shift $\Delta$ 88 nm und der Anteil der nutzbaren Fluoreszenz 79%.

Unter dem »Anteil der nutzbaren Fluoreszenz« versteht man den Prozentanteil des ursprünglich emittierten Fluoreszenzlichtes, der nicht durch Reabsorption verlorengeht.

Verfährt man analog zu Beispiel 1 mit den in der untenstehenden Tabelle aufgeführten Verbindungen, so erhält man die angegebenen Meßergebnisse.

Tabelle

| Bsp. | $R_1$ | $R_2$ | $R_3$ | $\Phi$ | $\Delta$ [nm] | Anteil der nutzbaren Fluoreszenz [%] |
|---|---|---|---|---|---|---|
| 2 | 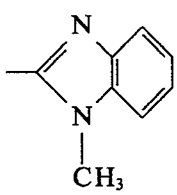 | H | H | 0,90 | 137 | 92 |
| 3 | 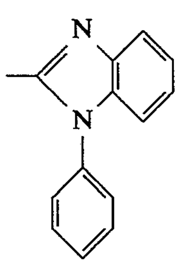 | H | H | 0,77 | 117 | 88 |
| 4 | 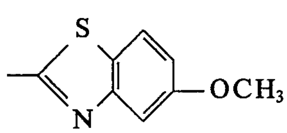 | H | H | 0,86 | 100 | 66 |

Zur Herstellung fester Lichtsammelsysteme werden die Verbindungen der Beispiele 1–4 zu 0,1 Gew.-% in handelsübliches Polyacrylat oder Polymethacrylat eingearbeitet, der gefärbte Kunststoff granuliert und zu Platten geeigneter Geometrie gepreßt.

**Patentansprüche**

1. Lichtsammelsystem, dadurch gekennzeichnet, daß es ein Cumarinderivat der Formel

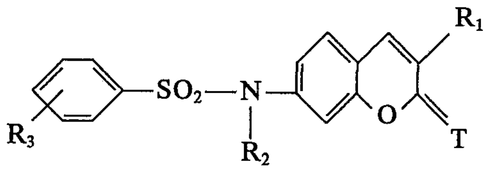

(I)

enthält, in der

T O oder $NR_4$, wobei
$R_4$ für Wasserstoff, gegebenenfalls substituiertes Alkyl oder gegebenenfalls substituiertes Aryl steht;

$R_1$ ein über ein C-Atom gebundener carbo- oder heterocyclischer sauerstofffreier 5- oder 6gliedriger Ring oder ein über ein N-Atom gebundener 5- oder 6gliedriger heterocyclischer Ring, der zu einer durch die Cumarin-N-Heterocyclus-Bindung gelegten Achse rotationsunsymmetrisch ist, wobei die genannten 5- oder 6gliedrigen Ringe nicht-ionogene Substituenten tragen können und an sie ein gegebenenfalls substituierter Benzol- oder ein gegebenenfalls substituierter Naphthalinring ankondensiert sein kann;

$R_2$ Wasserstoff, Alkyl, Cycloalkyl, Aralkyl, Aryl, wobei die genannten Kohlenwasserstoffreste substituiert sein können,

$R_3$ Wasserstoff, Alkyl, Cycloalkyl, Aralkyl, Aryl oder einen Rest $-OR_5$, $-SO_2R_5$, $-SO_2NHR_5$, $-NHCOR_5$, Halogen oder $COOR_5$ bezeichnen, wobei $R_5$ für Alkyl, Cycloalkyl, Aralkyl oder Aryl steht.

2. Lichtsammelsystem gemäß Anspruch 1, dadurch gekennzeichnet, daß es ein Cumarinderivat der Formel (I) enthält, bei dem $R_1$ für einen Pyrazolyl-, Imidazolyl-, Thiazolyl-, 1.2.4-Triazolyl-, 1.3.4-Thiadiazolyl-, Benzimidazolyl-, Benzthiazolyl-, Pyridinyl- oder einen vom Benzpyrimidon abgeleiteten Rest steht, wobei die genannten heterocyclischen Reste substituiert sein können.

3. Lichtsammelsystem, dadurch gekennzeichnet, daß es ein Cumarinderivat der Formel

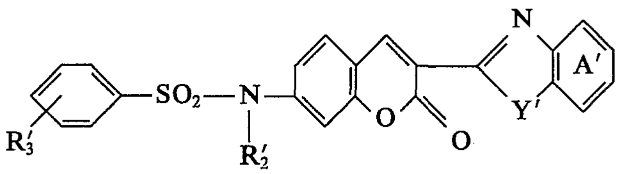

(III)

enthält,
in der

$R_2'$ Wasserstoff, $C_1-C_4$-Alkyl, insbesondere Methyl und Ethyl,

$R_3'$ Wasserstoff, $C_1-C_4$-Alkyl, insbesondere Methyl und Ethyl, $C_1-C_4$-Alkylsulfonyl, Phenylsulfonyl, $C_1-C_4$-Alkoxy, Phenoxy,

Y' $NR_6'$ oder S bezeichnen, wobei
$R_6'$ $C_1-C_4$-Alkyl, insbesondere Methyl, oder Phenyl bezeichnet und wobei der Ring

A' durch $C_1-C_4$-Alkyl, insbesondere Methyl, $C_1-C_4$-Alkoxy, insbesondere Methoxy und Halogen, insbesondere Chlor, substituiert sein kann.

4. Lichtsammelsystem, dadurch gekennzeichnet, daß es ein Cumarinderivat gemäß den Ansprüchen 1 bis 3 enthält, das in einer 0,05%igen Lösung, gemessen in einer Schichtdicke von 10 cm einen Anstieg der Transmission von 0% auf >90% bei einer Änderung der Wellenlänge von 25–100 nm, vorzugsweise 25–45 nm, aufweist.

5. Verwendung von Cumarinderivaten gemäß den Ansprüchen 1 bis 4 als Energiewandler in Lichtsammelsystemen.

## Claims

1. Light-collecting system, characterised in that it contains a coumarin derivative of the formula

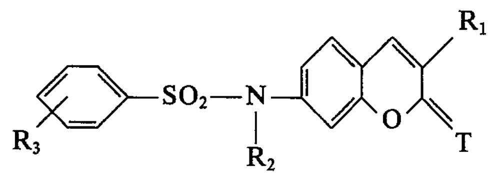

(I)

in which

T designates O or $NR_4$,
wherein
$R_4$ represents hydrogen, optionally substituted alkyl or optionally substituted aryl;
$R_1$ designates a carbocyclic or heterocyclic, oxygen-free 5-membered or 6-membered ring which is linked via a C atom or a 5-membered or 6-membered heterocyclic ring which is linked bia an N atom and which rotates unsymmetrically about an axis passing through the coumarin-N-heterocyclic ring linkage, it being possible for the 5-membered or 6-membered rings mentioned to carry non-ionic substituents and for an optionally substituted benzene ring or an optionally substituted naphthalene ring to be fused onto them;
$R_2$ designates hydrogen, alkyl, cycloalkyl, aralkyl or aryl, it being possible for the hydrocarbon radicals mentioned to be substituted, and
$R_3$ designates hydrogen, alkyl, cycloalkyl, aralkyl, aryl or a $-OR_5$, $-SO_2R_5$, $-SO_2NHR_5$, $-NHCOR_5$, halogen or $COOR_5$ radical,

wherein
$R_5$ represents alkyl, cycloalkyl, aralkyl or aryl.

2. Light-collecting system according to Claim 1, characterised in that it contains a coumarin derivative of the formula (I)
in which

$R_1$ represents a pyrazolyl, imidazolyl, thiazolyl, 1,2,4-triazolyl, 1,3,4-thiadiazolyl, benzimidazolyl, benzthiazolyl, pyridinyl radical or a radical derived from benzpyrimidone, it being possible for the heterocyclic radicals mentioned to be substituted.

3. Light-collecting system, characterised in that it contains a coumarin derivative of the formula

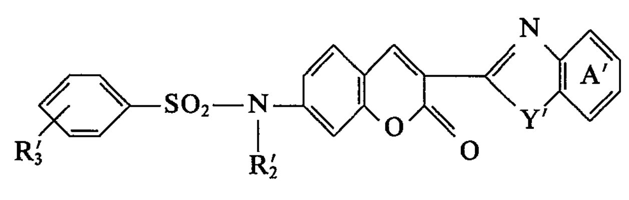

(III)

in which

$R_2'$ designates hydrogen or $C_1-C_4$-alkyl, in particular methyl or ethyl,
$R_3'$ designates hydrogen, $C_1-C_4$-alkyl, in particular methyl or ethyl, $C_1-C_4$-alkylsulphonyl, phenylsulphonyl, $C_1-C_4$-alkoxy or phenoxy and
Y' designates $NR_6'$ or S,

wherein

$R_6'$ designates $C_1-C_4$-alkyl, in particular methyl, or phenyl,

and wherein the ring A' can be substituted by $C_1-C_4$-alkyl, in particular methyl, $C_1-C_4$-alkoxy, in particular methoxy, or halogen, in particular chlorine.

4. Light-collecting system, characterised in that it contains a coumarin derivative according to Claims 1 to 3 which, in a 0.05% strength solution, exhibits a rise in transmission of 0% to >90% for a change in wavelength of 25—100 nm, preferably 25—45 nm, measured with a cell thickness of 10 cm.

5. Use of coumarine derivates according to Claims 1 to 4 as energy converters in ligth-collecting systems.

**Revendications**

1. Système collecteur de lumière, caractérisé en ce qu'il contient un dérivé de coumarine de formule:

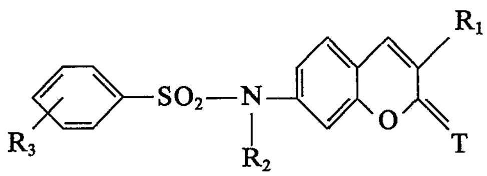

(I)

dans laquelle

T représente O ou $NR_4$,
 $R_4$ désignant l'hydrogène, un groupe alkyle éventuellement substitué ou un groupe aryle éventuellement substitué;
$R_1$ est un noyau pentagonal ou hexagonal non oxygéné carbocyclique ou hétérocyclique lié par l'intermédiaire d'un atome de carbone ou un noyau hétérocyclique pentagonal ou hexagonal lié par l'intermédiaire d'un atome d'azote, qui présente une dissymétrie de rotation par rapport à un axe passant par la liaison entre la coumarine et l'hétérocycle azoté, les noyaux pentagonaux ou hexagonaux en question pouvant porter des substituants non ionogènes et un noyau benzénique éventuellement substitué ou naphtalénique éventuellement substitué pouvant être condensé sur ces noyaux,
$R_2$ désigne l'hydrogène, un groupe alkyle, cycloalkyle, aralkyle, aryle, les restes hydrocarbonés mentionnés pouvant être substitués,
$R_3$ est l'hydrogène ou un groupe alkyle, cycloalkyle, aralkyle, aryle ou un reste $-OR_5$, $-SO_2R_5$, $-SO_2NHR_5$, $-NHCOR_5$, un halogène ou un reste $COOR_5$, $R_5$ représentant un radical alkyle, cycloalkyle, aralkyle ou aryle.

2. Système collecteur de lumière suivant la revendication 1, caractérisé en ce qu'il contient un dérivé de coumarine de formule (I) dans laquelle $R_1$ est un reste pyrazolyle, imidazolyle, thiazolyle, 1,2,4-triazolyle, 1,3,4-thiadiazolyle, benzimidazolyle, benzthiazolyle, pyridinyle ou un reste dérivé de la benzopyrimidone, les restes hétérocycliques mentionnés pouvant être substitués.

3. Système collecteur de lumière, caractérisé en ce qu'il contient un dérivé de coumarine de formule:

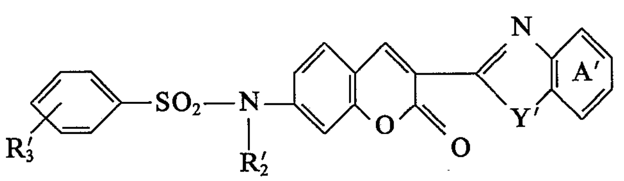

(III)

dans laquelle

$R_2'$ est l'hydrogène ou un groupe alkyle en $C_1$ à $C_4$, notamment méthyle et éthyle,
$R_3'$ est l'hydrogène, un groupe alkyle en $C_1$ à $C_4$, notamment méthyle et éthyle, alkylsulfonyle en $C_1$ à $C_4$, phénylsulfonyle, alkoxy en $C_1$ à $C_4$, phénoxy,
Y' représente un groupe $NR'_6$ ou S,
 $R'_6$ étant un radical alkyle en $C_1$ à $C_4$, notamment méthyle, ou le radical phényle et le noyau
A' pouvant être substitué par un substituant alkyle en $C_1$ à $C_4$, notamment méthyle, alkoxy en $C_1$ à $C_4$, notamment méthoxy et un halogène, en particulier le chlore.

4. Système collecteur de lumière, caractérisé en ce qu'il contient un dérivé de coumarine suivant les revendications 1 à 3 qui présente en solution à 0,05%, mesuré sous une épaisseur de couche de 10 cm, une montée de transmission de 0% à plus de 90% pour une variation de la longueur d'onde de 25 à 100 nm, de préférence de 25 à 45 nm.

5. Utilisation de dérivés de coumatine suivant les revendications 1 à 4 comme convertisseur d'énergie dans des systèmes collecteurs de lumière.